# EUROPEAN PATENT APPLICATION

(11) **EP 3 345 918 A1**
(43) Date of publication of application: **11.07.2018**
(21) Application number: 17305011.3
(22) Date of filing: 05.01.2017
(51) Int. Cl.: C07K 14/32, C07K 14/325, C12N 3/00, C12N 15/75, C12P 21/02, A01N 63/00, C12N 1/21

(54) **USE OF THE CPCR REGULATOR GENE FOR OBTAINING NEW RECOMBINANT STRAINS OF BACILLUS THURINGIENSIS WITH REDUCED SPORULATION CAPACITY**

(71) Applicant: Institut National De La Recherche Agronomique, 75007 Paris (FR); Institute of Plant Protection, Chinese Academy of Agricultural Sciences, Beijing 100193 (CN)
(72) Inventor: LERECLUS, Didier, 28260 Oulins (FR); SLAMTI, Leyla, 75015 Paris (FR); SONG, Fu Ping, Beijing 100193 (CN); DENG, Chao, Beijing 100193 (CN); ZHANG, Jie, Beijing 100193 (CN)
(74) Representative: Gevers & Orès

(57) **Abstract**

The present invention relates to the use of a *cpcR* regulator gene, which directs the expression of promoters of genes encoding Cry proteins, for reducing the sporulation of a strain of *Bacillus thuringiensis*, new recombinant strains of *Bacillus thuringiensis* and uses thereof as biopesticide.

## Description

### Field of the invention

The present invention relates to the use of a *cpcR* regulator gene for reducing the sporulation of a recombinant strain of *Bacillus thuringiensis*, a recombinant strain of *Bacillus thuringiensis* comprising the *cpcR* gene and uses thereof in particular as biopesticides.

### Background of the invention

### Bacteria and sporulation

*Bacillus* species are rod-shaped, endospore-forming aerobic or anaerobic, Gram-positive bacteria. The many species of the genus are able to live in every natural environment. Only one endospore is formed per cell. The spores are resistant to heat, cold, radiation, dessiccation, and disinfectants. At the onset of the sporulation, secondary metabolites are produced by *Bacillus sp..* These secondary metabolites may be: enzymes, antibiotics and insecticides.

*Bacillus larvae, Bacillus lentitmorbus, Bacillus popilliae, Bacillus sphaericus,* and *Bacillus thuringiensis* are pathogens of specific groups of insects (Bacillus, Medical Microbiology). Thus, some *bacillus* are used as the active ingredients of insecticides.

*Bacillus thuringiensis* (Bt) is a bacterium present in soil, which produces spores and synthetize crystals releasing proteins which are toxic to insects and classified as Cry1, -2, -3, -4,..., according to their peptide sequences (Crickmore *et al*., 1998). More particularly, Bt strains produce insecticidal crystals in the mother cell of sporulating bacteria which are used as biopesticides for controlling undesirable insects, such as Lepidoptera, Coleoptera and mosquitoes (Schnepf *et al*., 1998). At the end of sporulation, the crystal is found alongside the spore. The promoters of genes encoding these crystal toxins are known. They are for example the promoters controlling the expression of genes *cry1, cry2, cry3, cry4* (Agaisse *et al*., 1995; Deng *et al*., 2014). The promoters of the *cry1, cry2 and cry4* genes are recognized by RNA polymerases associated to the specific sigma factors, Sigma E or Sigma K; the promoters of the *cry3* genes are recognized by RNA polymerase associated to the vegetative sigma factor, Sigma A.

### Cry toxins

Cry toxins have specific activities against insect species of the orders Lepidoptera, Diptera, Coleoptera, Hymenoptera and against nematodes. When insects ingest crystals, their alkaline digestive tracts denature the crystals, making them soluble and thus amenable to being cut with proteases found in the insect gut, which liberate the toxin from the crystal (Schnepf *et al*., 1998). The Cry toxin is then inserted into the insect gut cell membrane, paralyzing the digestive tract and forming a pore. The insect stops eating and starves to death (Schnepf *et al*., 1998).

### Bt-based biopesticides

Bt is successfully used as a biopesticide for more than 50 years (Bravo *et al*., 2011 and Sanahuja *et al*., 2011). However, the efficiency of these Bt-based biopesticides is assessed to only a few days on the leaf surface. Indeed, the chemistry of the leaf surface, proteases and sunlight contribute to the degradation of Cry proteins.

Today, all the Bt-based biopesticides used in the world consist of a mixture of spores and crystals. However, toxins present in crystals are rapidly destroyed because they are released in the external medium at the end of the sporulation process. Further, the dissemination of bacterial spores may be detrimental for Human and environment.

It remains a need to provide safer Bt-based biopesticides wherein the stability of the Cry toxins is improved.

### Detailed description of the invention

In order to solve these problems, the inventors studied a Bt strain, called LM1212 that presents the unique ability to differentiate into crystal-producers or spore-formers. Transcriptional analysis suggested that the parasporal crystal phenotype results from a new type of cell differentiation associated with a novel regulation mode of *cry* gene expression (Deng *et al*., 2015). As specified above, all biopesticides known until now consist of a mixture of spores and crystals. The characterization of the unusual LM1212 strain, in which the crystal is not produced in the mother cell of sporulating cells, but only in a subpopulation of non-sporulating cells, expanded inventor's understanding of the parasporal crystal phenotype in Bt. Thereby, the inventors have identified surprisingly and unexpectedly a unique regulatory sequence gene present in this LM1212 and called "crystal producing cells regulator" (*cpcR*). They showed that the introduction of this *cpcR* gene in a typical Bt strain drastically allows to reduce sporulation rate.

The inventors have characterized this regulator gene and its nucleotide sequence, which comprises the sequence SEQ ID NO: 1.

Thus, this CpcR regulator directs the expression of the *cry* gene promoters in *Bacillus thuringiensis* LM1212.

The inventors have also shown that the use of this regulator allows the expression of heterologous *cry* genes, as the *cry1Ab* gene encoding a lepidopteran active toxin, and the production of crystal inclusions in non sporulating Bt cells.

Moreover, the introduction of the *cpcR* gene in a typical Bt strain drastically reduced its sporulation rate while maintaining or increasing the expression of *cry* genes. The results obtained by the inventors indicate that this expression system allows the production of insecticidal Cry proteins in a Spo⁻ genetic background. Thus, the invention provides safer Bt-based biopesticides by preventing the dissemination of bacterial spores. Moreover, the production and the stability of the Cry toxins in dedicated bacterial cells are improved since the toxins are protected from the environmental degradation, such as UV irradiation.

Accordingly, the present invention relates to the use of the *cpcR* regulator gene of the sequence SEQ ID NO: 1 for reducing the sporulation of a recombinant strain of *Bacillus thuringiensis.*

The nucleotide sequence SEQ ID NO: 1 is:

ATGAATCCTAATATACTTGTGATAAATGGTAATACGAAGAATCAAGAATTT
ATTCAAAATCTCCTGATTATAAAGAAATTCATGATTGATACGGTAAATGATG
GTATAGAAGGAATTATTCGCTTTCAAAAGAAAGTGTACGATCTAGTTATACT
AGATGTTATGTCACCTAATTTAGATGGATTTAGTATATGTAAAATTATAAGA
TCACAATCTAAAGTGCCAATTATCATGTTATCTACAATAAAGGACGAAAGT
ATTGAGATTAAAGGATTTCAATTTGGTATTGATGACTTTATTACTCTACCAT
GTTCAGTTGAGCTATTTTATTATAGGATAGAAGCCATTCTACGAGGACGAAA
TTCAACTGATTCATTGTCATTAATACAGTTTCAAGAAATATCACTAAATCCT
GATTCGTATATAGTGTATCTTAATGGGCAGAAGAAAAAGCTAACGACAAAA
GAATTTGATATGCTACATATATTTTTAAGAAATCCAGGAAAGTATTATCTA
GAGAATTTTTGCTGAATCAAGTGTGGGGATATGATTATTATGGAGATCCACG
AGTCATAGATGCGTATATAAAAAAACTACGCAAAAAGTTAAGTATTCCGTA
TATAAAAACAATAACAGGCGTTGGTTATAAACTAGACACATAA

"Recombinant strain" in the present invention means that one or more fragments of genes or one or more entire genes is/are inserted by genetic engineering, in a parental strain.

"Reducing the sporulation" in the present invention means that the rate of sporulation of the recombinant strain of Bt of the invention is reduced at least 2 fold preferably at least 5 fold, more preferably at least 10 fold, even more preferably at least 15 fold compared to the sporulation rate of the parental Bt strain.

In order to characterize the *cpcR* regulator gene, first the inventors determined a DNA fragment from LM1212, responsible for the activation of a promoter of a gene encoding a Cry protein in the LM1212. Then, they reduced this DNA fragment until identifying the gene responsible for this effect. After identification, the inventors validated that *cpcR* is responsible for a) the activation of *cry* gene expression in the LM1212 strain and b) the reduction of the sporulation rate. Finally, the inventors tested the use of the *cpcR* in a recombinant strain of Bt and obtained a reduction of the sporulation rate and an increase of the production of Cry1Ab. These results confirm the interest to use the *cpcR* gene in a recombinant strain of Bt and the use of this recombinant strain of Bt as new biopesticide.

The *cpcR* regulator directs the expression of several promoters of genes encoding Cry and Cyt proteins, said promoters having the consensus sequence SEQ ID NO:2.

The nucleotide sequence SEQ ID NO: 2 is:
X₁TGAAX₂AAAAX₃X₄X₅X₆CAX₇X₈AX₉ATTTX₁₀CX₁₁TCX₁₂X₁₃X₁₄X₁₅X₁₆TX ₁₇X₁₈AX₁₉ATGTX₂₀X₂₁TX₂₂GX₂₃TAX₂₄AX₂₅TX₂₆X₂₇X₂₇X₂₉AX₃₀X₃₁TX₃₂X₃₃, with:
   X₁=A or G; X₂ =C or T; X₃=A or T; X₄=A, T or G; X₅=A, C or T; X₆=A or G; X₇=C or T; X₈=A or C X₉=A, T or G; X₁₀=A or C; X₁₁= A or C; X₁₂=A, C or T_{;} X₁₃=A, G or T; X₁₄=A or C; X₁₅=A, G or T; X₁₆=A or G; X₁₇=A or T; X₁₈=A, C or T; X₁₉=C or T; X₂₀=A or C; X₂₁=A, C or G; X₂₂=A or T; X₂₃= C or T; X₂₄=G or T; X₂₅=C or T; X₂₆=G or T; X₂₇=A or G; X₂₈=A or T; X₂₉=A, G or T; X₃₀=C, G or T; X₃₁=A or G; X₃₂=A or G; X₃₃=C or T.

Preferably, the promoter activated by CpcR, is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID: 6.

More preferably, the promoter activated by CpcR is P₃₅.

According to the invention, genes encoding the proteins activated by CpcR in the LM1212 strain, are preferably *cry* genes, more preferably *cry32Va1, cry41Ca1, cry45Ba1, cry74Aa1, cry32Wa1* and *cry35-like*, even more preferably *cry32Wa1* and *cry35-like.*

According to the invention, genes encoding proteins, in particular toxins, produced by the recombinant strain of *Bacillus thuringiensis* are preferably *cry* genes or *cyt* genes.

Preferably, the *cry* and *cyt* genes encoding toxins produced by the recombinant strain of the invention under the control of CpcR are:
1) *cry* genes encoding toxins active against crop pests, specifically lepidopteran and coleopteran insects. These *cry* genes belong for example to the classes *cry1, cry2, cry3, cry8* or *cry9.*
2) *cry* genes encoding toxins active against insect vectors of human diseases, specifically dipteran insects as mosquitoes and simuli. These *cry* genes belong for example to the classes *cry4* and *cry11.*
3) *cry* genes encoding toxins active against nematodes. These *cry* genes belong for example to the classes *cry5, cry6, cry14* and *cry21.*
4) *cyt* genes encoding toxins active against insect vectors of human diseases, specifically dipteran insects as mosquitoes and simuli. These *cyt* genes belong for example to the classes *cyt1* and *cyt2.*

More preferably, the genes encoding toxins produced by the recombinant strain of the invention under the control of CpcR are *cry1, cry2, cry3, cry4, cry5, cry 6 cry8, cry9, cry11, cry14, cry21, cyt1* or *cyt2* genes.

The activity spectrum of the Cry and Cyt toxins against insect pests is described in: Van Frankenhuizen, K., *et al*., 2009. The activity spectrum of the Cry toxins against nematodes is described in: Wei *et al*., 2003.

More preferably, the toxin produced by the recombinant strain of the invention is Cry1Ab, a lepidopteran active toxin, encoded by the *cry1Ab* gene.

The inventors showed that the expression of the *cpcR* gene allows the expression of heterologous *cry* genes, such as the *cry1Ab* gene, and the production of crystal inclusions in non-sporulating Bt cells.

The present invention also relates to a recombinant strain of *Bacillus thuringiensis*, which is characterized in that it comprises:
a) At least one gene encoding Cry and/or Cyt toxins,
b) At least one promoter having the sequence SEQ ID NO: 2, allowing the expression of said at least one gene encoding Cry and/or Cyt toxins, and,
c) A *cpcR* regulator gene of sequence SEQ ID NO: 1, which directs the expression of said at least one promoter.

The main feature of this recombinant strain, in the present invention, is its reduced sporulation rate in comparison with the parental strain.

According to the invention, this bacterium may be used living or dead. Indeed, the viability of the bacteria is not required to ensure the insecticidal or the nematicidal activity of this recombinant strain of *Bacillus thuringiensis.* Since CpcR negatively affects sporulation, the number of viable spores will be reduced or even abolished.

Preferably, the parental strain of *Bacillus thuringiensis* is the *Bacillus thuringiensis kurstaki* HD73 (type strain of the serotype 3a, 3b, 3c).

Preferably, the promoter present in the recombinant strain of *Bacillus thuringiensis* is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID: 6.

More preferably, the promoter present in the recombinant strain of *Bacillus thuringiensis* is P₃₅.

According to the invention, genes encoding the toxins in the recombinant strain of *Bacillus thuringiensis* are preferably *cry* or *cyt* genes, more preferably *cry1, cry2, cry3, cry4, cry5, cry6, cry8, cry9, cry11, cry 14, cry 21, cyt1* or *cyt2 genes*, more preferably, *cry1, cry2, cry3, cry8 or cry9* genes.

Preferably, the toxins produced by the recombinant strain of the invention are Cry1 and Cry2 toxins.

More preferably, the toxin produced by the recombinant strain of the invention is Cry1Ab, a lepidopteran active toxin, encoded by the *cry1Ab* gene.

The present invention also relates to the use of the recombinant strain of *Bacillus thuringiensis* as described above as biopesticide.

"Biopesticide" in the present invention refers to pesticides derived from natural materials, such as bacteria, for example, in the present invention. This general term "biopesticide" includes the term "biocide" which is more used in a context of controlling vectors, such as vectors that transmit pathogens responsible for mammalian diseases.

In a particular embodiment, the recombinant strain of *Bacillus thuringiensis* is used for protecting cultures. The "biopesticide" producing CpcR-regulated Cry proteins will be used for controlling crop pests, specifically lepidopteran insects such as those belonging to the families *Tortricidae, Noctuidae* and *Pyralidae* which damage crops like cotton, cabbage, corn, soybean, grapevine and any other extensive or gardener cultures.

Preferably, the toxins produced by the recombinant strain of the invention for protecting cultures are: Cry1, Cry2, Cry3, Cry 8 and Cry9 toxins.

In another particular embodiment, the recombinant strain of *Bacillus thuringiensis* is used to control vectors, especially vectors that transmit pathogens responsible for mammalian diseases, preferably human diseases, such as insect vectors, in particular mosquitoes or simuli. In such a particular embodiment, the recombinant strain of *Bacillus thuringiensis* will harbor CpcR-regulated *cry* genes encoding toxins specifically active against dipteran insects.

Preferably, the toxins produced by the recombinant strain of the invention in the use to control vectors are toxins belonging, for example, to the classes Cyt1, Cry4 and Cry11.

In another particular embodiment, the recombinant strain of *Bacillus thuringiensis* is used to control nematodes, especially those responsible for mammalian diseases, preferably human diseases. In such a particular embodiment, the recombinant strain of *Bacillus thuringiensis* will harbor CpcR-regulated *cry* genes encoding toxins specifically active against nematodes.

Preferably, the toxins produced by the recombinant strain of the invention in the use to control nematodes are toxins belonging, for example, to the classes Cry5, Cry6, Cry 14 and Cry21.

The present invention also relates to a method for obtaining a recombinant strain of *Bacillus thuringiensis* as described above, comprising the steps of introducing in said strain both:
1- a genetic construction comprising at least one gene encoding a toxin under the control of a promoter having the sequence SEQ ID NO: 2, and
2- an expression system comprising the CpcR regulator of the sequence SEQ ID NO: 1.

The two steps of this method can be performed in the order as defined above or in the reverse order but also simultaneously by introducing a construct carrying the three cited elements.

Preferably, the parental strain of *Bacillus thuringiensis* is the *Bacillus thuringiensis kurstaki* HD73.

Preferably, the promoter having the sequence SEQ ID NO: 2 is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID: 6.

More preferably, said promoter is P₃₅ of sequence SEQ ID NO: 2.

Preferably, the genes encoding a toxin are *cry1, cry2, cry3, cry 4, cry5, cry6, cry8, cry9, cry11, cry14, cry2₁* or *cyt* genes, more preferably *cry1, cry2, cry3, cry8* or *cry9 genes.*

More preferably, the gene encoding a toxin is *cry1Ab* gene.

The promoter which directs the transcription of the *cpcR* gene is any promoter functional in Bt. The choice of the more appropriate promoter may depend in particular on the type of expression (i.e. constitutive or inducible) that one wishes to obtain. Promoters may be constitutive promoters, *i.e.* promoters which are active in cells and under most environmental conditions, or cell specific promoters which are active only or mainly in certain cell types, and inducible promoters that are activated by physical or chemical stimuli.

The genetic construction will be done on Bacillus plasmids based on the replicons pBC16, pHT315 or pHT73 and carrying a *cry* gene downstream from a CpcR-regulated promoter.

The genetic construct can be introduced in a strain of *Bacillus thuringiensis* by electroporation (Lereclus *et al*., 1989; Bone *et al.* 1989; Koehler *et al.* 1994; Mahillon *et al*., 1999; Peng *et al* 2009), by using recombination procedure as previously described (Lereclus *et al*., 1992), or by heterogramic conjugation (Trieu-Cuot *et al*., 1987).

In addition to the above features described, the invention further comprises other features which will emerge from the following description, which refers to examples illustrating the present invention, as well as to the appended figures.
**Figure 1** shows the construction of a *Bacillus thuringiensis* strain.
**Figure 2** shows the characterization of the LM1212 DNA fragment carrying the *cpcR* gene activating transcription from the P₃₅ promoter.
**Figure 3** shows the expression of *cry1Ab* under the control of CpcR and P₃₅ in a *kurstaki* strain. A) Plasmids pHT16-18ΩP35'-*cry1Ab* and pHT-1c. B) Phase-contrast microscopy of the strain *kurstaki* HD73 transformed with plasmids pHT16-18ΩP35'-*cry1Ab* and pHT-1c. The arrows indicate some crystal inclusions.

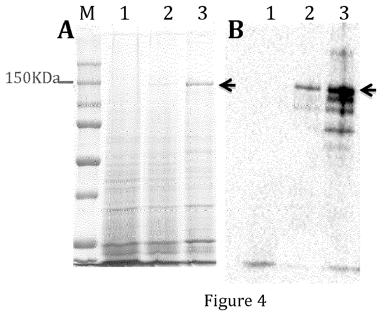

**Figure 4** shows the analysis of a Cry1Ab toxin produced in a *kurstaki* strain under the control of CpcR and P₃₅. A) SDS-Page. M corresponds to molecular weight markers. 1. 20 µL of crude extract from colonies of the strain *kurstaki* HD73 (pHT16-18, pHT304). 2. 20 µL of crude extract from colonies of the strain *kurstaki* HD73 (pHT16-18ΩP35'-*cry1Ab*, pHT304). 3. 20 µL of crude extract from colonies of the strain *kurstaki* HD73 (pHT16-18Ω35'-*cry1Ab*, pHT-1c). The crude extracts were prepared in the same way and the use of 20 µL of crude extract means that the same amount of each preparation is loaded on the SDS-Page. B) Western blotting of lanes 1, 2 and 3 with antisera against Cry1Ab.

### EXAMPLE 1: CONSTRUCTION OF A BACILLUS THURINGIENSIS STRAIN TO SCREEN FOR THE REGULATOR OF THE EXPRESSION FROM THE P₃₅ PROMOTER

The previous results obtained by the inventors suggested that, in the LM1212 strain, a regulation system limits the production of toxins to the subpopulation of non-sporulating cells. Thus, to screen the LM1212 genes responsible for the regulation of the P₃₅ promoter, the promoter directing the expression of the *cry* gene, the inventors introduced a reporting system into a *Bacillus thuringiensis* strain, as described thereafter.

### 1) Materials and Methods

The Bt strain *kurstaki* HD73 Cry is chosen to clone the LM1212 genes responsible for the activation of the P₃₅ promoter and for the negative effect on sporulation.

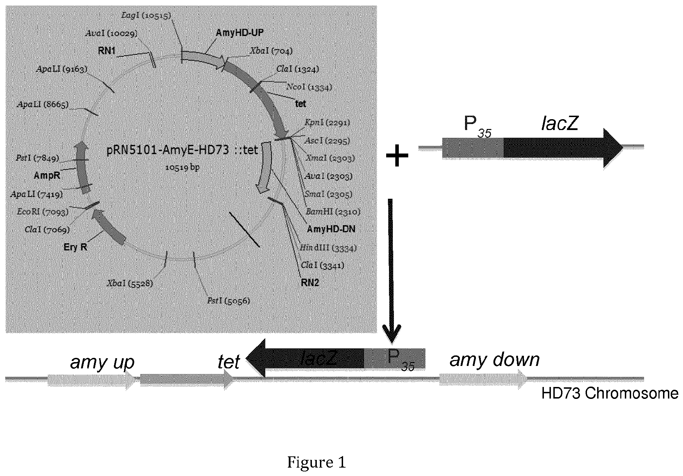

The thermosensitive plasmid pRN5101-AmyE-HD73::tet is first constructed following the same procedure as described in Verplaetse *et al.,* 2015, except that the antibiotic resistance cassette confers resistance to tetracycline instead of spectinomycin and that the *amyE* flanking regions used for homologous recombination were amplified using Bt HD73 genomic DNA instead of Bt 407. A P_{35'}-*lac*Z transcriptional fusion is then cloned between the *amy* up and *amy* down fragments cloned from the *kurstaki* strain. The *P*_{*35*'}*-lacZ* fusion is then introduced at the *amyE* locus of the *kurstaki* HD73 chromosome by homologous recombination (Figure 1).

### 2) Results

The resulting strain is designated HD73 Cry [amyE::P_{35'}-*lacZ*].

### EXAMPLE 2: CHARACTERIZATION OF THE LM1212 GENES ACTIVATING TRANSCRIPTION FROM THE P₃₅ PROMOTER

### i. Localization of the genes responsible for the expression from the P₃₅ promoter on the DNA of LM1212 strain

### 1) Materials and Methods

The DNA fragment ORF28-ORF32 from plasmid PLM248 of strain LM1212 is cloned into plasmid pHT304 (Arantes, O. et al., 1991) and the resulting plasmid is called pHT-1a. pHT-1a is then introduced into the Bt strain HD73 Cry [*amyE*::*P*_{*35*'}-*lacZ*] and the recombinant clones are isolated on HCT plates (0.7% casein hydrolysate, 0.5% tryptone, 0.68% KH2 PO4, 0.012% MgSO4_7H2 O, 0.00022% MnSO4_4H2 0, 0.0014% ZnSO4_7H2 O, 0.008% ferric ammonium citrate, 0.018% CaCl2_4H2 O, 0.3% glucose, pH 7.2) (Lecadet *et al*., 1980; Verplaetse *et al*., 2016) containing yeast extract (0,05%), glucose 0.3% and X-Gal (100 µg / mL).

### 2) Results

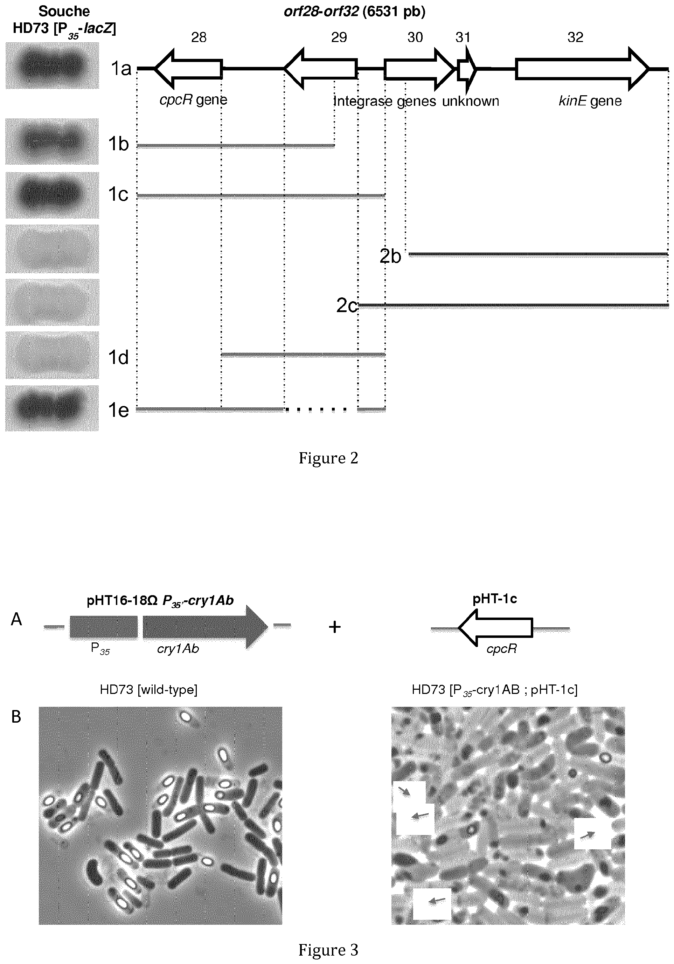

The parental strain gives white colonies. In sharp contrast, the strain harboring plasmid pHT-1a gives blue colonies (Figure 2). This result indicates that the genes responsible for the expression from the P₃₅ promoter are located on the DNA fragment la corresponding to LM1212 ORF28 to 32.

### ii. Identification of the genes involved in the transcriptional activity

### 1) Materials and Methods

The DNA region ORF28 to 32 is sub-cloned into the pHT304 plasmid and the resulting plasmids are transformed into the Bt strain HD73 Cry [*amyE*::*P_{35'}*-*lacZ*] (figure 2).

### 2) Results

It appears that the ORF28 encoding a transcriptional regulator is sufficient to activate P₃₅-directed transcription. However, this activity is obtained if a short DNA fragment located just upstream of the ORF29 is fused upstream from ORF28 (Fragment le, figure 2). ORF28 encodes a transcriptional regulator belonging to the family of response regulators, suggesting that it should act as a two-component system in association with a histidine kinase. Such a kinase may be encoded by ORF32. However, results show that ORF28 alone is able to activate the P₃₅ promoter. *In silico* analysis of these genes (ORF28 and 32) indicates that ORF28 is unique and is not found in the available sequences of the bacteria of the *B. cereus* group (about 100 genomes). In contrast, ORF32 is highly conserved among all the bacteria of the *B. cereus* group, including the LM1212.

Thus, ORF28 may function as a response regulator activated by the histidine kinase naturally present in the *kurstaki* HD73 strain.

### iii. Effect of the plasmid pHT-1c on the sporulation of the bacteria

### 1) Materials and Methods

pHT-1c, which corresponds to the plasmid pHT304 carrying the gene ORF28 and a promoter region, is introduced in Bt strain HD73 Cry [*amyE*::P_{35'}*-lacZ*].

### 2) Results

The introduction of pHT-1c in Bt strain HD73 Cry⁻ [*amyE*::*P*_{35'}-*lac*Z] negatively affects sporulation by a 10-fold factor (Table 1).

**Table 1. Effect of the DNA fragment 1 c carrying the cpcR gene on the sporulation of B. thuringiensis stain HD73. The results are the mean of two independent experiments. ^{a}The bacteria were grown in liquid HCT medium (containing Yeast Extract 0.05% and Glucose 0.3%) at 30°C, 48 h after inoculation, aliquots were heated at 80°C for 12 mins. The cells were plated and CFUs corresponding to heat-resistant spores were then counted.**

| **Strains** | **Heat-resistant spores (CFU/ml)^{a}** |
|---|---|
| HD73 *amyE*::*Pcry35-lacZ* (pHT304) | 5,25E+08 (±5,25E+07) |
| HD73 *amyE*::*Pcry35-lacZ* (pHT304-1c) | 4,99E+07 (±9,42E+06) |

### iv. Conclusion

These data indicate that the gene corresponding to ORF28 encodes the regulator (designated as CpcR) responsible for two functions: i) the activation of *cry* gene expression in the LM1212 strain, and ii) the reduction of the sporulation rate.

The results also suggest that ORF28 functions as a two-component system associated with a kinase existing in all the Bt strains. This gene is responsible for the specific phenotype of the LM1212 strain, which is to differentiate into crystal-producers or spore-formers.

### EXAMPLE 3: USE OF THE CPCR REGULATOR TO PRODUCE CRY1Ab IN NON-SPORULATING BT CELLS

In order to validate the use of CpcR in a Bt strain for reducing its sporulation while maintaining toxin production, the inventors produced recombinant strains of Bt and analyzed the sporulation rate of this recombinant strain and the production of the toxin Cry1Ab.

### 1 ) Materials and Methods

a) The *cry1Ab* gene, a typical sporulation-dependent *cry* gene is PCR amplified from strain *kurstaki* HD1 (the biopesticide *kurstaki* HD1 Dipel®) and is cloned downstream from the P₃₅ promoter into plasmid pHT16-18 (Lereclus *et al*., 1992). The resulting plasmid, pHT16-18Ω P35'-*cry1Ab* (*i.e.* the plasmid pHT16-18 carrying the *cry1Ab* gene from the *kurstaki* HD1 Dipel® strain under the control of the P₃₅ promoter) is transformed into Bt strain HD73 Cry⁻. The resulting strain was then transformed with the plasmid pHT-1c carrying the *cpcR* gene. The bacteria were plated on HCT agar plates for 48 h at 30°C and examined in phase-contrast microscopy (Figure 3).
b) The proteins produced by these recombinant strains are analyzed by western blotting with antisera against the Cry1Ab toxin (Figure 4).

### 2) Results

a) The results show the production of typical crystal inclusion in non-sporulating cells of the *kurstaki* strain harboring plasmids pHT16-180ΩP_{35'}-cry1Ab and pHT-1c. Moreover, the sporulation rate of the recombinant strain carrying pHT16-180ΩP_{35'}-*cry1Ab* and pHT-1c is significantly lower than that of the wild-type strain.
b) The production of Cry1Ab is strongly increased in the *kurstaki* strain harboring the *cpcR* gene and the P_{35'}*-cry1Ab* fusion (Panels A and B, Lanes 3).
   The faint band of Cry1Ab observed in lanes 2 results from a low CpcR-independent expression of *cry1Ab.*

These results show that the invention provides a new range of biopesticides by the way of production of insecticidal Cry toxins in non-sporulating Bt strains.

### REFERENCES

Agaisse, H. et al., J. Bacteriol. 1995. 177: 6027-6032.
Arantes, O. et al., Gene 1991. 108: 115-119.
Bone, EJ. et al., FEMS Microbiol Lett. 1989. 49(2-3):171-177.
Bravo A. et al., Insect Biochem Mol Biol. 2011. 41: 423 -43 1.
Crickmore, N. et al., Microbiol. Mol. Biol. Rev. 1998. 62: 807-813.
Deng, C. et al., Toxins 2014. 6: 2194-2209.
Deng C. et al., The ISME Journal, 2015. 9: 286-296.
Fromm et al., Nature 1990. 319:791-793.
Koehler TM, et al. J Bacteriol. 1994. 176(3):586-595.
Lecadet M et al., Microbiology. 1980. 121: 1: 203-212.
Lereclus, D. et al., FEMS Microbiol. Lett. 1989. 60: 211-217.
Lereclus, D. et al., Nature Biotechnology 1992. 10: 418-421.
Lereclus, D. et al., Mol. Microbiol. 1992. 7: 35-46.
Mahillon, J. et al., Eynard & Teissie (Eds.) Springer Lab. Manual 1999. 242-252.
Peng, D. H., et al. J. Appl. Microbial. 2009. 106:1849-1858
Sanahuja G. et al., Plant Biotechnol Journal. 2011. 9:283-300.
Schnepf, E. et al., Microbiol. Mol. Biol. Rev. 1998. 62: 775-806.
Trieu-Cuot, P. et al., FEMS Microbiol Lett 1987. 48: 289-294.
Van Frankenhuizen, K. et al., J. Invertebr. Pathol. 2009. 101 : 1-16.
Verplaetse, E. et al., MBio 2015. 6: e136008-15.
Verplaetse E. et al., Res Microbiol. 2016. doi: 10.1016/j.resmic.2016.03.006.
Wei J-Z. et al., PNAS 2003. 100(5):2760-2765.

## Claims

1. Use of the *cpcR* regulator gene of the sequence SEQ ID NO: 1 for reducing the sporulation of a recombinant strain of *Bacillus thuringiensis.*

2. The use of the *cpcR* gene as claimed in claim 1, wherein said CpcR regulator directs the expression of promoters of genes encoding Cry and Cyt proteins, said promoters having the sequence SEQ ID NO: 2 as follows:
X₁TGAAX₂AAAAX₃X₄X₅X₆CAX₇X₈AX₉ATTTX₁₀CX₁₁TCX₁₂X₁₃X₁₄X₁₅X₁₆ TX₁₇X₁₈AX₁₉ATGTX₂₀X₂₁TX₂₂GX₂₃TAX₂₄AX₂₅TX₂₆X₂₇X₂₈X₂₉AX₃₀X₃₁TX₃₂ X₃₃, with: X₁=A or G; X₂ =C or T; X₃=A or T; X₄=A, T or G; X₅=A, C or T; X₆=A or G; X₇=C or T; X₈=A or C X₉=A, T or G; X₁₀=A or C; X₁₁= A or C; X_{F2}=A, C or T; X₁₃=A, G or T; X₁₄=A or C; X₁₅=A, G or T; X₁₆=A or G; X₁₇=A or T; X₁₈=A, C or T; X₁₉=C or T; X₂₀=A or C;X₂₁=A, C or G; X₂₂=A or T; X₂₃= C or T; X₂₄=G or T; X₂₅=C or T; X₂₆=G or T; X₂₇=A or G; X₂₈=A or T; X₂₉=A, G or T; X₃₀=C, G or T; X₃₁=A or G; X₃₂=A or G; X₃₃=C or T.

3. The use of the *cpcR* gene as claimed in claim 2, **characterized in that** the promoter is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID NO: 6.

4. The use of the *cpcR* gene as claimed in any one of claims 1 to 3, **characterized in that** the genes encoding toxins are *cry* genes or *cyt* genes.

5. The use of the *cpcR* gene as claimed in any one of claims 1 to 4, **characterized in that** the strain of *Bacillus* comprises *cry1, cry2, cry3, cry4, cry5, cry6, cry8, cry9, cry11, cry14, cry21, cyt1* or *cyt2* genes.

6. A recombinant strain of *Bacillus thuringiensis* **characterized in that** it comprises:
a) At least one gene encoding Cry and/or Cyt toxins,
b) At least one promoter having the sequence SEQ ID NO: 2, allowing the expression of said at least one gene encoding Cry and/or Cyt toxins, and,
c) A *cpcR* regulator gene of sequence SEQ ID NO: 1, which directs the expression of said at least one promoter.

7. The recombinant strain of *Bacillus thuringiensis* as claimed in claim 6, **characterized in that** the promoter is selected from the group consisting of: P₃₂ of the sequence SEQ ID NO: 3, P₄₁ of the sequence SEQ ID NO: 4, P₃₅ of the sequence SEQ ID NO: 5, P₄₅ of the sequence SEQ ID NO: 6.

8. The recombinant strain of *Bacillus thuringiensis* as claimed in claim 6 or in claim 7, **characterized in that** the genes encoding toxins are *cry* genes or *cyt* genes.

9. The recombinant strain of *Bacillus thuringiensis* as claimed in any one of claims 6 to 8, **characterized in that** the strain of *Bacillus* comprises *cry1, cry2, cry3, cry4, cry5, cry6, cry8, cry9, cry14, cry21, cyt1* or *cyt2* genes.

10. Use of a recombinant strain of *Bacillus thuringiensis* as claimed in anyone of claims 6-9 as biopesticide.

11. The use of the recombinant strain of *Bacillus thuringiensis* as claimed in claim 10 for protecting cultures.

12. The use of the recombinant strain of *Bacillus thuringiensis* as claimed in claim 10 to control vectors, especially vectors that transmit pathogens responsible for mammalian diseases, such as insect vectors, in particular mosquitoes or simuli.

13. The use of the recombinant strain of *Bacillus thuringiensis* as claimed in claim 10 to control nematodes, especially those responsible for mammalian diseases.

14. A method for obtaining a recombinant strain of *Bacillus thuringiensis*, comprising the steps of introducing in said strain both:
1- a genetic construction comprising at least one gene encoding a toxin under the control of a promoter having the sequence SEQ ID NO: 2, and
2- an expression system comprising the CpcR regulator of the sequence SEQ ID NO: 1.


SEQUENCE LISTING

<110>  INSTITUT NATIONAL DE LA RECHERCHE AGRONOMIQUE
       INSTITUTE OF PLANT PROTECTION, CHINESE ACADEMY OF
       AGRICULTURAL SCIENCES
       LERECLUS, Didier
       SLAMTI, Leyla
       SONG, Fu Ping
       DENG, Chao
       ZHANG, Jie

<120>  Use of the CpcR regulator gene for obtaining new recombinant
       strains of Bacillus thuringiensis with reduced sporulation
       capacity

<130>  F539/186

<160>  6

<170>  PatentIn version 3.5

<210>  1
<211>  663
<212>  DNA
<213>  Bacillus thuringiensis

<400>  1
atgaatccta atatacttgt gataaatggt aatacgaaga atcaagaatt tattcaaaat      60

ctcctgatta taaagaaatt catgattgat acggtaaatg atggtataga aggaattatt     120

cgctttcaaa agaaagtgta cgatctagtt atactagatg ttatgtcacc taatttagat     180

ggatttagta tatgtaaaat tataagatca caatctaaag tgccaattat catgttatct     240

acaataaagg acgaaagtat tgagattaaa ggatttcaat ttggtattga tgactttatt     300

actctaccat gttcagttga gctattttat tataggatag aagccattct acgaggacga     360

aattcaactg attcattgtc attaatacag tttcaagaaa tatcactaaa tcctgattcg     420

tatatagtgt atcttaatgg gcagaagaaa aagctaacga caaaagaatt tgatatgcta     480

catatatttt taagaaatcc agggaaagta ttatctagag aatttttgct gaatcaagtg     540

tggggatatg attattatgg agatccacga gtcatagatg cgtatataaa aaaactacgc     600

aaaaagttaa gtattccgta tataaaaaca ataacaggcg ttggttataa actagacaca     660

taa                                                                   663

<210>  2
<211>  65
<212>  DNA
<213>  Artificial Sequence

<220>
<223>  Promoter activated by CpcR consensus sequence

<220>
<221>  Variant

<222>  (1)..(1)
<223>  Replace = a

<220>
<221>  Variant
<222>  (6)..(6)
<223>  Replace = t

<220>
<221>  Variant
<222>  (11)..(11)
<223>  Replace = t

<220>
<221>  Variant
<222>  (12)..(12)
<223>  Replace = t or g

<220>
<221>  Variant
<222>  (13)..(13)
<223>  Replace = c or t

<220>
<221>  Variant
<222>  (14)..(14)
<223>  Replace = a

<220>
<221>  Variant
<222>  (17)..(17)
<223>  Replace = t

<220>
<221>  Variant
<222>  (18)..(18)
<223>  Replace = c

<220>
<221>  Variant
<222>  (20)..(20)
<223>  Replace = t or g

<220>
<221>  Variant
<222>  (25)..(25)
<223>  Replace = a

<220>
<221>  Variant
<222>  (27)..(27)
<223>  Replace = c

<220>
<221>  Variant
<222>  (30)..(30)
<223>  Replace = c or t

<220>
<221>  Variant
<222>  (31)..(31)
<223>  Replace = a or g

<220>
<221>  Variant
<222>  (32)..(32)
<223>  Replace = c

<220>
<221>  Variant
<222>  (33)..(33)
<223>  Replace = g or t

<220>
<221>  Variant
<222>  (34)..(34)
<223>  Replace = g

<220>
<221>  Variant
<222>  (36)..(36)
<223>  Replace = t

<220>
<221>  Variant
<222>  (37)..(37)
<223>  Replace = c or t

<220>
<221>  Variant
<222>  (39)..(39)
<223>  Replace = t

<220>
<221>  Variant
<222>  (44)..(44)
<223>  Replace = c

<220>
<221>  Variant
<222>  (45)..(45)
<223>  Replace = c or g

<220>
<221>  Variant
<222>  (47)..(47)
<223>  Replace = a

<220>
<221>  Variant
<222>  (49)..(49)
<223>  Replace = t

<220>
<221>  Variant
<222>  (52)..(52)
<223>  Replace = t

<220>
<221>  Variant
<222>  (54)..(54)
<223>  Replace = c

<220>
<221>  Variant
<222>  (56)..(56)

<223>  Replace = t

<220>
<221>  Variant
<222>  (57)..(57)
<223>  Replace = g

<220>
<221>  Variant
<222>  (58)..(58)
<223>  Replace = t

<220>
<221>  Variant
<222>  (59)..(59)
<223>  Replace = g or t

<220>
<221>  Variant
<222>  (61)..(61)
<223>  Replace = g or t

<220>
<221>  Variant
<222>  (62)..(62)
<223>  Replace = a

<220>
<221>  Variant
<222>  (64)..(64)
<223>  Replace = g

<220>
<221>  Variant
<222>  (65)..(65)
<223>  Replace = t

<400>  2
gtgaataaaa aaagcacaaa atttccatca tcagttaaca tgtaattgct agattgaaaa      60

cgtac                                                                  65


<210>  3
<211>  65
<212>  DNA
<213>  Bacillus thuringiensis

<400>  3
gtgaacaaaa agtacatcaa atttccatcc tcggtttaca tgtacttgct atattggaga      60

ggtac                                                                  65


<210>  4
<211>  65
<212>  DNA
<213>  Bacillus thuringiensis

<400>  4
gtgaacaaaa aacacatcag atttccatct gagatttaca tgtacttgct atattggtaa      60

ggtac                                                                  65

<210>  5
<211>  65
<212>  DNA
<213>  Bacillus thuringiensis

<400>  5
gtgaataaaa taagcacaat atttccctca tcagttcata tgtcattgct agactgaaga      60

tgtat                                                                  65


<210>  6
<211>  65
<212>  DNA
<213>  Bacillus thuringiensis

<400>  6
atgaataaaa tttgcacaag atttacatca actataaaca tgtagtagtt atatttaata      60

catgt                                                                  65